# DEMANDE DE BREVET EUROPEEN

(11) **EP 4 427 741 A1**
(43) Date de publication de la demande: **11.09.2024**
(21) Numéro de dépôt: 24161348.8
(22) Date de dépôt: 05.03.2024
(51) Int. Cl.: A61K 9/16, C07C 15/00, A61K 9/14, A61K 31/00

(54) **COMPOSITION COMPRENANT AU MOINS UNE DIBENZO-ALPHA-PYRONE NATURELLE OU SYNTHÉTIQUE COMME SUBSTANCE ACTIVE ET SON PROCÉDÉ DE FABRICATION**

(30) Priorité: 06.03.2023 BE 202305164
(71) Demandeur: Eleonor, 1410 Waterloo (BE); Tilman, 5377 Baillonville (BE)
(72) Inventeur: Loira-Pastoriza, Cristina, 1120 Bruxelles (BE); Priem, Fabian, 1410 Waterloo (BE); Dierckxsens, Yvan, 3090 Overijse (BE)
(74) Mandataire: Gevers Patents

(57) **Abrégé**

La présente invention se rapporte à une composition comprenant au moins une dibenzo-α-pyrone naturelle ou synthétique comme substance active, à son procédé de fabrication et à son utilisation.

## Description

La présente invention se rapporte à une composition comprenant au moins une dibenzo-α-pyrone naturelle ou synthétique comme substance active, à son procédé de fabrication et à son utilisation.

Au sens de la présente invention, les termes « dibenzo-α-pyrone » et « dibenzo-alpha-pyrone » sont des synonymes.

Selon l'invention, une dibenzo-α-pyrone naturelle ou synthétique est un composé dont la structure chimique de base est la suivante (Formule générale (I)): 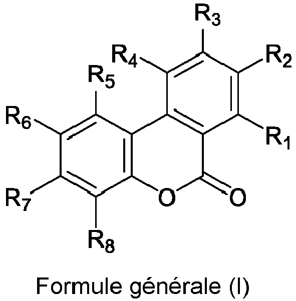 dans laquelle,
R₁ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₂ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₃ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₄ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₅ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₆ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₇ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₈ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4.

En particulier, selon un mode de réalisation suivant la présente invention, R₁ représente préférentiellement un hydrogène, un alkyle en C₁₋₄, OR', OSOsR', C(=O)R', OC(=O)R', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Préférentiellement, R₁ représente un hydrogène, OR', un halogène, un O-glucoside, ou un C-glucoside; R' représentant un hydrogène ou un alkyle en C₁₋₄.

Plus préférentiellement, R₁ représente un hydrogène, ou OR' ; R' représentant un hydrogène ou un alkyle en C₁₋₄.

Plus préférentiellement, R₁ représente un hydrogène, OH ou OCH₃.

En particulier, selon un mode de réalisation suivant la présente invention, R₂ représente préférentiellement un hydrogène, un alkyle en C₁₋₄, OR', OSOsR', C(=O)R', OC(=O)R', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Préférentiellement, R₂ représente un hydrogène, un alkyle en C₁₋₄, OR', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Plus préférentiellement, R₂ représente un hydrogène, un alkyle en C₁₋₄, OR', ou un O-glucoside; ledit alkyle en C₁₋₄ étant éventuellement substitué par un ou plusieurs hydroxy; R' représentant un hydrogène ou un alkyle en C₁₋₄.

Plus préférentiellement, R₂ représente un hydrogène, CH₃, OH, OCH₃ ou un O-glucoside.

En particulier, selon un mode de réalisation suivant la présente invention, R₃ représente préférentiellement un hydrogène, un alkyle en C₁₋₄, C(=O)OR', OR', OSOsR', C(=O)R', OC(=O)R', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Préférentiellement, R₃ représente un hydrogène, un alkyle en C₁₋₄, C(=O)OR', OR', OSOsR', C(=O)R', OC(=O)R', un halogène, un O-glucoside, ou un C-glucoside; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C₁₋₄.

Plus préférentiellement, R₃ représente un hydrogène, OR', OSOsR', C(=O)R', un halogène, un O-glucoside, ou un C-glucoside; ledit O-dihydrobenzofurane étant éventuellement substitué par un ou plusieurs groupements choisi parmi OR' ou CH₂OR'; R' représentant un hydrogène ou un alkyle en C₁₋₄.

Plus préférentiellement, R₃ représente un hydrogène, OH, OCH₃, C(=O)C_{1-4 alkyl}, OSOsH ou un O-dihydrobenzofurane substitué par un ou plusieurs groupements choisi parmi OH ou CH₂OH.

En particulier, selon un mode de réalisation suivant la présente invention, R₄ représente préférentiellement un hydrogène, un alkyle en C₁₋₄, OR', OSOsH, C(=O)R', OC(=O)R', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Préférentiellement, R₄ représente un hydrogène, OR', un halogène, un O-glucoside, ou un C-glucoside; R' représentant un hydrogène ou un alkyle en C₁₋₄.

Plus préférentiellement, R₄ représente un hydrogène, ou OR'; R' représentant un hydrogène ou un alkyle en C₁₋₄.

Plus préférentiellement, R₄ représente un hydrogène, OH ou OCH₃,
En particulier, selon un mode de réalisation suivant la présente invention, R₅ représente préférentiellement un hydrogène, un alkyle en C₁₋₄, OR', OSOsH, C(=O)R', OC(=O)R', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Préférentiellement, R₅ représente un hydrogène, un alkyle en C₁₋₄, OR', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Plus préférentiellement, R₅ représente un hydrogène, un alkyle en C₁₋₄, ou OR'; ledit alkyle en C₁₋₄ étant éventuellement substitué par un ou plusieurs hydroxy; R' représentant un hydrogène ou un alkyle en C₁₋₄.

Plus préférentiellement, R₅ représente un hydrogène, CH₃, CH₂OH, ou OH.

En particulier, selon un mode de réalisation suivant la présente invention, R₅ représente préférentiellement un hydrogène, un alkyle en C₁₋₄, OR', OSOsH, C(=O)R', OC(=O)R', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Préférentiellement, R₆ représente un hydrogène, OR', un halogène, un O-glucoside, ou un C-glucoside; R' représentant un hydrogène ou un alkyle en C₁₋₄.

Plus préférentiellement, R₆ représente un hydrogène, OR', ou un halogène, R' représentant un hydrogène ou un alkyle en C₁₋₄.

Plus préférentiellement, R₆ représente un hydrogène, OH, OCH₃ ou Cl.

En particulier, selon un mode de réalisation suivant la présente invention, R₇ représente préférentiellement un hydrogène, un alkyle en C₁₋₄, OR', OSOsH, C(=O)R', OC(=O)R', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Préférentiellement, R₇ représente un hydrogène, OR', OSOsH, un halogène, un O-glucoside, ou un C-glucoside; R' représentant un hydrogène ou un alkyle en C₁₋₄.

Plus préférentiellement, R₇ représente un hydrogène, OR', OSOsH, ou un O-glucoside; R' représentant un hydrogène ou un alkyle en C1-4.

Plus préférentiellement, R₇ représente un hydrogène, OH, OCH₃, un O-glucoside, ou OSOsH.

En particulier, selon un mode de réalisation suivant la présente invention, R₈ représente préférentiellement un hydrogène, un alkyle en C₁₋₄, OR', OSOsH, C(=O)R', OC(=O)R', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Préférentiellement, R₈ représente un hydrogène, un alkyle en C₁₋₄, OR', un halogène, un O-glucoside, ou un C-glucoside; ledit alkyle en C₁₋₄ étant éventuellement substitué par un ou plusieurs hydroxy; R' représentant un hydrogène ou un alkyle en C₁₋₄.

Plus préférentiellement, R₈ représente un hydrogène, un alkyle en C₁₋₄, OR', ou un halogène, ledit alkyle en C₁₋₄ étant éventuellement substitué par un ou plusieurs hydroxy; R' représentant un hydrogène ou un alkyle en C₁₋₄.

Plus préférentiellement, R₈ représente un hydrogène, CH₃, OH, OCH₃ ou Cl.

Selon des modes de réalisation préférés suivant la présente invention, la dibenzo-α-pyrone est préférentiellement un composé dont la structure chimique de base est choisie parmi l'une des suivantes (Formules générales (II) à (VIII)) : 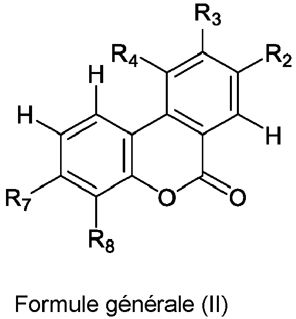 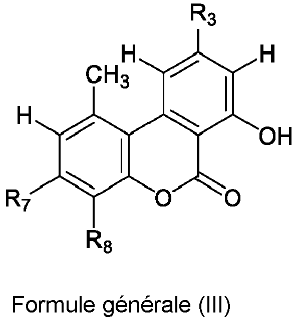 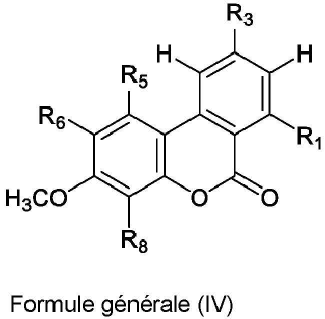 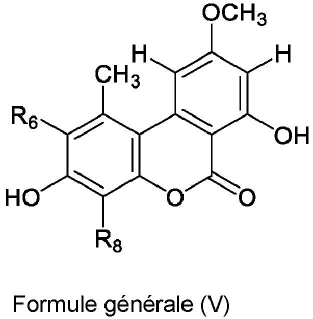 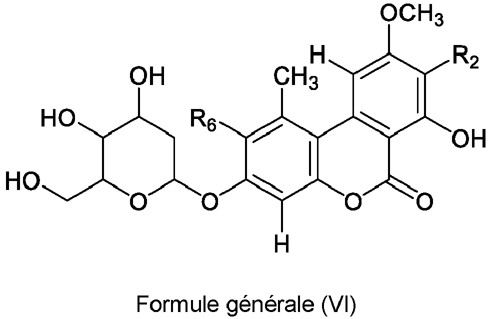 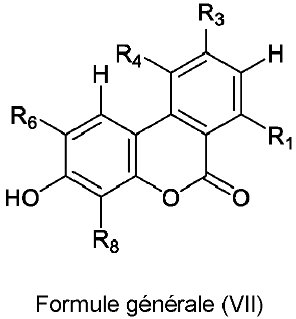 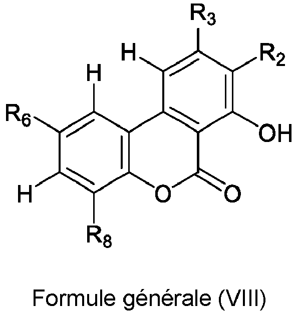 dans lesquelles,
R₁, R₂, R₃, R₄, R₅, R₆, R₇, et R₈ sont tels que définis précédemment pour la formule générale (I).

Selon un mode de réalisation préféré suivant la présente invention, ladite dibenzo-α-pyrone selon la formule (II) est un composé choisi parmi l'urolithine A, l'isourolithine A, l'urolithine B, l'urolithine C, l'urolithine D, l'urolithine E, l'urolithine M-5, l'urolithine M-6, l'urolithine M-7, l'isourolithine B, l'urolithine A glucuronide, l'urolithine B glucuronide, la 8-Omethylurolithine A, la 8,9-di-O-methylurolithine C, et la 8,9-di-O-methylurolithine D.

Préférentiellement, suivant la présente invention, ladite dibenzo-α-pyrone selon la formule (II) est un composé choisi parmi l'urolithine A, l'isourolithine A, l'urolithine B, l'isourolithine B, l'urolithine C, l'urolithine D, l'urolithine E, l'urolithine A glucuronide, et l'urolithine B glucuronide.

Plus préférentiellement, suivant la présente invention, ladite dibenzo-α-pyrone selon la formule (II) est un composé choisi parmi l'urolithine A, l'isourolithine A, et l'urolithine B.

Selon un mode de réalisation préféré suivant la présente invention, ladite dibenzo-α-pyrone selon la formule (III) est un composé choisi parmi alternariol, l'éther 9-méthylique d'alternariol, l'éther 9-méthylique du 3-O-sulfate d'alternariol, le 9-O-sulfate d'alternariol, et l'éther 9-méthylique du 4-hydroxyalternariol.

Selon un mode de réalisation préféré suivant la présente invention, ladite dibenzo-α-pyrone selon la formule (IV) est un composé choisi parmi la graphislactone A, la graphislactone B, la graphislactone C, la graphislactone E, et la graphislactone F, la graphislactone G, et la graphislactone H.

Selon un mode de réalisation préféré suivant la présente invention, ladite dibenzo-α-pyrone selon la formule (V) est un composé choisi parmi le palmariol A et le palmariol B.

Selon un mode de réalisation préféré suivant la présente invention, ladite dibenzo-α-pyrone selon la formule (VI) est un composé choisi parmi la lysilactone A, la lysilactone B, et la lysilactone C.

Selon un mode de réalisation préféré suivant la présente invention, ladite dibenzo-α-pyrone selon la formule (VII) est un composé choisi parmi l'altertenuol, le fasciculiferol.

Selon un mode de réalisation préféré suivant la présente invention, ladite dibenzo-α-pyrone selon la formule (VIII) est un composé choisi parmi la sabilactone et la murylactone.

Le terme « alkyle », utilisé seul ou en combinaison, est défini comme étant un radical dérivé d'un alcane comprenant, sauf indications contraires, de 1 à 4 atomes de carbone. Par exemple, un alkyle en C_{F-G} définit un radical alkyle non ramifié ou ramifié contenant de F à G atomes de carbone, par exemple un alkyle en C₁₋₄ définit un alkyle non ramifié ou ramifié contenant de 1 à 4 carbones tel que par exemple, un méthyle, un éthyle, un 1-propyle, un 2-propyle, un 1-butyle, un 2-butyle, un 2-méthyl-1-propyle. Un groupe alkyle peut être ramifié ou non-ramifié. La chaîne alkyle ramifiée ou non ramifiée peut être liée à n'importe quelle position dans la mesure où un composé stable est obtenu.

Le terme « halogène », utilisé seul ou en combinaison est défini comme désignant tous les halogènes, c'est-à-dire, les groupements chloro (Cl), bromo (Br), fluoro (F) et iodo (I).

Le terme « glucoside » utilisé seul ou en combination, est défini comme étant un composé dans lequel au moins un saccharide est lié à un autre groupement fonctionnel par une liaison glucosidique. La chaîne glucosidique peut comprendre de 1 à 4 saccharides.

Le terme « liaison glucosidique », utilisé seul ou en combinaison, est défini comme étant une liaison formée entre un groupement hemiacétal d'un saccharide et un groupement fonctionnel d'un composé. Ledit groupement fonctionnel peut être un groupement OH (O-glucoside) ou un groupement -CR₁R₂R₃ (C-glucoside).

Les termes « O-glucoside acylé » ou « C-glucoside acylé », utilisés seuls ou en combinaison, sont définis comme étant un composé dans lequel au moins un hydroxyle de la chaîne glucosidique est estérifiée par un acide organique, tel que, par exemple, l'acide acétique, l'acide benzoïque, les acides cinnamiques (tels que l'acide caféique, l'acide férulique, ou l'acide p-coumarique) et/ou l'acide phénylpropanoïque.

Les termes « O-glucoside sulfaté » ou « C-glucoside sulfaté », utilisés seuls ou en combinaison, sont définis comme étant un composé dans lequel au moins un hydroxyle de la chaîne glucosidique est estérifiée par de l'acide sulfurique.

Au sens de la présente invention, les termes « dibenzo-α-pyrone naturelle ou synthétique » désignent tant les dibenzo-α-pyrones d'origine naturelle que les dibenzo-α-pyrones de synthèse, mais aussi tous les dérivés des dibenzo-α-pyrones.

Au sens de la présente invention, une substance active, un principe actif ou un ingrédient actif désigne une substance bioactive (un composé bioactif) qui entre dans la composition d'un médicament ou d'un complément alimentaire ou de toute autre forme administrable à un être vivant et qui confère à ce médicament, à ce complément alimentaire ou à toute autre forme administrable à un être vivant ses propriétés thérapeutiques ou préventives.

Il est reconnu que les dibenzo-α-pyrones naturelles ou synthétiques sont des composés très peu voire totalement insolubles dans l'eau dans laquelle ils ne se dispersent d'ailleurs que faiblement ou pas du tout, ces composés présentant dès lors de très faibles biodisponibilités / bioaccessibilités.

Pourtant, malgré leur faible biodisponibilité / bioaccessibilité (c'est-à-dire malgré la faible fraction de la dose administrée qui atteint effectivement la circulation sanguine sous forme inchangée) mais aussi malgré leur faible solubilité et/ou malgré leur faible dispersion notamment dans le milieu intestinal, les effets positifs des dibenzo-α-pyrones naturelles ou synthétiques sur diverses pathologies en font des molécules d'intérêt en vue d'une administration à l'être humain et/ou pour un usage vétérinaire.

Malheureusement, il apparait que les formulations / compositions actuelles comprenant des dibenzo-α-pyrones naturelles ou synthétiques sont peu appropriées en raison de leur trop faible voire de leur non-solubilité et/ou en raison de leur trop faible voire de leur non dispersion en phase aqueuse et/ou en raison du faible relargage de ces composés au départ de ces formulations / compositions, ce qui se traduit finalement par une faible biodisponibilité / bioaccessibilité de ces molécules d'intérêt. Notons qu'il apparait aussi que les procédés actuels de fabrication de ces formulations sont contraignants et difficiles à mettre en oeuvre.

Par les termes « dispersion en phase aqueuse (en milieu aqueux) », il est entendu, au sens de la présente invention, un système composé de deux phases dans lequel l'une des deux phases, appelée phase dispersée, est finement répartie dans l'autre, appelée phase dispersante. Cette dispersion peut être moléculaire (solution), colloïdale (dispersion de particules submicroniques) ou plus grossière (dispersion de particules supérieures au µm). Plus particulièrement, selon l'invention, les termes « dispersion en phase aqueuse » désignent les suspensions, constituées d'une phase solide dispersée dans une phase aqueuse (liquide).

Au sens de la présente invention, le terme « solubilité », désigne la capacité d'une substance, appelée soluté, à se dissoudre dans une autre substance, appelée solvant, pour former un mélange homogène appelé solution.

L'invention a pour but de pallier les inconvénients de l'état de la technique en procurant (1) une composition comprenant au moins une dibenzo-α-pyrone naturelle ou synthétique comme substance active dont la solubilité et/ou la dispersion en phase aqueuse (milieu aqueux) est augmentée de telle sorte que la biodisponibilité / la bioaccessibilité de ces composés soit significativement augmentée et (2) un procédé de fabrication d'une telle composition qui soit facile à mettre en oeuvre, qui soit flexible, qui soit économiquement rentable et qui assure que ladite au moins une dibenzo-α-pyrone naturelle ou synthétique comme substance active soit présente et répartie de façon homogène dans la composition finale obtenue.

Par ailleurs, l'invention entend procurer une composition qui est stable au cours du temps, c'est-à-dire qui conserve ses propriétés en termes de solubilité et/ou de dispersion de ladite au moins une dibenzo-α-pyrone naturelle ou synthétique comme substance active et qui conserve ses propriétés en termes de taux de relargage de ce composé au cours du temps au départ d'une composition (formulation) selon l'invention, ceci en particulier en phase aqueuse et plus particulièrement dans le milieu intestinal.

Pour résoudre au moins partiellement ces problèmes, il est prévu suivant l'invention, une composition sous forme d'un extrudat thermoformé, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, comprenant au moins une dibenzo-α-pyrone naturelle ou synthétique comme substance active et au moins un support naturel ou synthétique.

Le terme « éventuellement » employé ci-dessus signifie « optionnellement » ou encore « en particulier », ce qui ne limite aucunement la composition selon l'invention à l'une des formes sous laquelle elle pourrait être conditionnée.

Avantageusement, selon l'invention, ledit extrudat thermoformé est sous une forme solide, en particulier sous une forme solide friable / cassante.

Préférentiellement, selon l'invention, ledit extrudat thermoformé est friable / cassant. En d'autres termes, préférentiellement, selon l'invention, ladite composition se présente sous forme d'un extrudat thermoformé friable / cassant.

Par les termes « extrudat thermoformé friable / cassant », il est entendu, au sens de la présente invention, un extrudat qui se désagrège facilement, qui peut être réduit facilement en poudre et/ou en de petits fragments / morceaux.

Par le terme « support », il est entendu, au sens de la présente invention, un composé ou une molécule, en particulier un composé ou une molécule qui permet la dispersion / la solubilisation / l'intégration en son sein de l'actif (la dibenzo-α-pyrone) de telle sorte à former un mélange / une dispersion homogène.

Avantageusement, selon l'invention, ledit au moins un support naturel ou synthétique est un support thermoplastique, c'est-à-dire un support ayant la propriété de se ramollir lorsqu'il est chauffé suffisamment, mais qui, se refroidissant, redevient dur, friable / cassant.

Avantageusement, selon l'invention, ledit au moins un support naturel ou synthétique peut être un polymère, en particulier un polymère thermoplastique.

Avantageusement, selon invention, le terme « support » désigne les molécules / composés de type polymère (par exemple les polysaccharides) ou non (par exemples les acides aminés).

Avantageusement, selon invention, ledit support naturel ou synthétique est choisi dans le groupe constitué de acides aminés, des peptides et des polypeptides naturels ou synthétiques, des protéines naturelles ou synthétiques, des saccharides naturels ou synthétiques, des lipides naturels ou synthétiques, des polymères synthétiques, de l'urée, de leurs dérivés et de leurs mélanges.

Avantageusement, selon l'invention, ledit support naturel ou synthétique est un acide aminé choisi dans le groupe constitué des acides aminés suivants : acide aspartique, acide cystéique, acide muramique, acide pyroglutamique, alanine, arginine, asparagine, aspartate, citrulline, cystéine, glutamate, glutamine, glycine, histidine, homocystéine, homosérine, hydroxyproline, isovaline, isoleucine, leucine, lysine, méthionine, methylalanine, norleucine, norvaline, ornithine, phénylalanine, proline, sarcosine, sérine, taurine, thréonine, tryptophane, tyrosine, valine, leurs dérivés et leurs mélanges. Cette liste est non exhaustive.

Avantageusement, selon l'invention, ledit support naturel ou synthétique est un peptide ou un polypeptide choisi dans le groupe constitué de l'aspartame, de la carnosine, du gluthation, des peptides de collagène, des hydrolysats de protéines, de leurs dérivés et leurs mélanges. Cette liste est non exhaustive.

Au sens de la présente invention, le terme « protéine » désigne tant les protéines hydrolysées ou non, par exemple les protéines végétales ou animales hydrolysées ou non.

Avantageusement, selon l'invention, ledit support naturel ou synthétique est une protéine (hydrolysée ou non) choisie dans le groupe constitué des protéines suivantes : les caséines, les glycoprotéines, les collagènes, les hydrolysats de collagènes, les gélatines, les protéines de pois, les protéines de soja, les protéines de blé, les protéines de citrouille, les protéines de noix, les protéines de riz, les protéines de noisette, les protéines de lait, les protéines de petit pois, les protéines de lentilles, les protéines d'amandes, les protéines de pistaches, les protéines de chia, les protéines de chanvre, les protéines de lin, les protéines de courge, les protéines de sésame, les protéines de pavot, les protéines de lupin, les protéines de tournesol, les protéines d'avoine, les protéines de millet, les protéines d'orge, les protéines de seigle, les protéines de maïs, la zéine, leurs dérivés et leurs mélanges.

A titre d'exemple, lorsque ladite au moins une protéine est du collagène ou de la gélatine, il peut s'agir de collagène ou de gélatine d'origine animale (poisson, porc, boeuf, ...).

En particulier et avantageusement selon l'invention, lesdits hydrolysats de collagène sont des hydrolysats de collagène n'ayant pas / ne présentant pas la capacité de gélifier, c'est-à-dire des hydrolysats de collagène non gélifiants.

Le terme « protéine » désigne une macromolécule constituée de différents acides aminés qui sont liés entre eux. La protéine est l'élément le plus commun et l'unité moléculaire de base de tout être vivant. Les protéines sont caractérisées par la longueur de leur chaîne moléculaire.

Le terme « acide aminé » désigne un acide carboxylique qui possède également un groupe fonctionnel amine et le terme « peptide » désigne un polymère d'acides-aminés.

Le terme « collagène » désigne une protéine composée de trois chaînes alpha polypeptidiques associées. Ces chaînes sont reliées par des liaisons hydrogène entre l'hydroxylysine et l'hydroxyproline et des liaisons covalentes.

Le terme « gélatine » désigne du collagène qui a été modifié, les chaînes moléculaires restant de préférence suffisamment longues pour assurer les propriétés fonctionnelles recherchées avec la gélatine (viscosité, tenue de la gelée).

Les termes « peptides de collagène » désignent de petits groupements ordonnés d'acides aminés issus de la fragmentation de la molécule de collagène. En fonction de la technique d'hydrolyse utilisée, ces groupements sont plus ou moins important et plus ou moins actifs.

Les termes « collagène hydrolysé » indiquent que le collagène a été hydrolysé, notamment pour le rendre plus assimilable.

Préférentiellement, selon l'invention, lesdits collagènes et/ou lesdits hydrolysats de collagène et/ou lesdites gélatines et/ou lesdits hydrolysats de protéines (ou lesdites protéines hydrolysées) présentent un poids moléculaire compris entre 50 et 300000 Da, de préférence entre 100 et 275000 Da, de préférence entre 150 et 250000 Da, de préférence entre 200 et 225000 Da, de préférence entre 250 et 200000 Da, de préférence entre 300 et 175000 Da, de préférence entre 350 et 150000 Da, de préférence entre 400 et 125000 Da, de préférence entre 450 et 100000 Da, de préférence entre 500 et 75000 Da, de préférence entre 550 et 50000 Da, de préférence entre 600 et 40000 Da, de préférence entre 650 et 30000 Da, de préférence entre 700 et 20000 Da, de préférence entre 750 et 10000 Da, de préférence entre 800 et 9000 Da, de préférence entre 850 et 8000 Da, de préférence entre 900 et 7000 Da, de préférence entre 950 et 6000 Da, de préférence entre 1000 et 5000 Da, de préférence entre 1050 et 4000 Da, de préférence entre 1100 et 3000 Da, de préférence entre 1150 et 2000 Da, de préférence entre 1200 et 1000 Da.

Avantageusement, selon l'invention, lesdits collagènes et/ou lesdits hydrolysats de collagène et/ou lesdites gélatines présentent un poids moléculaire compris entre 1000 et 300000 Da, de préférence entre 1500 et 150000 Da, de préférence entre 2000 et 60000 Da.

De préférence, selon l'invention, lesdits collagènes et/ou lesdits hydrolysats de collagène présentent un poids moléculaire égal à 50 Da ou égal à 100 Da ou égal à 150 Da ou égal à 200 Da ou égal à 250 Da ou égal à 300 Da ou égal à 350 Da ou égal à 400 Da ou égal à 450 Da ou égal à 500 Da ou égal à 550 Da ou égal à 600 Da ou égal à 650 Da ou égal à 700 Da ou égal à 750 Da ou égal à 800 Da ou égal à 850 Da ou égal à 900 ou égal à 950 Da ou égal à 1000 Da ou égal à 1100 Da ou égal à 1200 ou égal à 1300 Da ou égal à 1400 Da ou égal à 1500 Da ou égal à 1600 Da ou égal à 1700 Da ou égal à 1800 Da ou égal à 1900 Da ou égal à 2000 Da ou égal à 2500 Da ou égal à 3000 Da ou égal à 3500 Da ou égal à 4000 Da ou égal à 4500 Da ou égal à 5000 Da ou égal à 5500 Da ou égal à 6000 Da ou égal à 6500 Da ou égal à 7000 Da ou égal à 7500 Da ou égal à 8000 Da ou égal à 8500 Da ou égal à 9000 Da ou égal à 9500 Da ou égal à 10000 Da ou égal à 12500 Da ou égal à 15000 Da ou égal à 17500 Da ou égal à 20000 Da ou égal à 22500 Da ou égal à 25000 Da ou égal à 27500 Da ou égal à 30000 Da ou égal à 32500 Da ou égal à 35000 Da ou égal à 37500 Da ou égal à 40000 Da ou égal à 42500 Da ou égal à 45000 Da ou égal à 47500 Da ou égal à 50000 Da ou égal à 55000 Da ou égal à 60000 Da ou égal à 65000 Da ou égal à 70000 Da ou égal à 75000 Da ou égal à 80000 Da ou égal à 85000 Da ou égal à 90000 Da ou égal à 100000 Da ou égal à 110000 Da ou égal à 120000 Da ou égal à 130000 Da ou égal à 140000 Da ou égal à 150000 Da ou égal à 160000 Da ou égal à 170000 Da ou égal à 180000 Da ou égal à 190000 da ou égal à 200000 Da ou égal à 210000 Da ou égal à 220000 Da ou égal à 230000 Da ou égal à 240000 Da ou égal à 250000 Da ou égal à 260000 Da ou égal à 270000 Da ou égal à 280000 Da ou égal à 290000 Da ou égal à 300000 Da.

Suivant un mode de réalisation selon l'invention, lorsque ladite au moins une protéine est du collagène, ce dernier présente un poids moléculaire compris entre 900 et 7000 Da, de préférence un poids moléculaire compris entre 950 et 5000 Da, préférentiellement un poids moléculaire compris entre 1000 et 3000 Da.

Suivant un mode de réalisation selon l'invention, lorsque ladite au moins une protéine est du collagène, ce dernier présente un poids moléculaire égal à 2000 Da ou égal à 3000 Da ou égal à 5000 Da ou égal à 50000 Da.

Suivant un mode de réalisation selon l'invention, lorsque ladite au moins une protéine est de la gélatine, cette dernière présente un poids moléculaire compris entre 900 et 6000 Da, de préférence un poids moléculaire compris entre 950 et 5000 Da, préférentiellement un poids moléculaire compris entre 1000 et 3000 Da.

Suivant un mode de réalisation selon l'invention, lorsque ladite au moins une protéine est de la gélatine, cette dernière présente un poids moléculaire égal à 2000 Da ou égal à 3000 Da ou égal à 5000 Da ou égal à 50000 Da.

Suivant un mode de réalisation selon l'invention, lorsque ladite au moins une protéine est un collagène hydrolysé ou un peptide de collagène, ce dernier présente un poids moléculaire compris entre 900 et 6000 Da, de préférence un poids moléculaire compris entre 950 et 5000 Da, préférentiellement un poids moléculaire compris entre 1000 et 3000 Da.

Suivant un mode de réalisation selon l'invention, lorsque ladite au moins une protéine est un collagène hydrolysé ou un peptide de collagène, ce dernier présente un poids moléculaire égal à 2000 Da ou égal à 3000 Da ou égal à 5000 Da ou égal à 50000 Da.

Avantageusement, selon l'invention, ledit support naturel ou synthétique est saccharide choisi dans le groupe constitué des saccharides suivants : le raffinose, le rhamminose, le mannose, le glucose, le galactose, le fructose, le rhamnose, le saccharose, le stachyose, le verbascose, le tréhalose, le lactose, le lactulose, le maltose, l'érythritol, le mannitol, le sorbitol, le xylitol, les cyclodextrines (l'α-cyclodextrine, la β-cyclodextrine, la γ-cyclodextrine), l'isomalt, le xylane, les alginates, les amidons (l'amidon de maïs, l'amidon de pomme de terre, l'amidon de riz, l'amidon de blé, l'amidon de sarrasin, l'amidon de châtaigne, l'amidon de patate douce, l'amidon de pois, ou encore l'amidon de manioc), les amidons prégélatinisés, les amidons modifiés, les fibres solubles ou insolubles (fibres de Konjac, les fibres d'acacia, les fibres d'inuline, les fibres de chicorée ou encore les fibres d'arabinoxylane), les carraghénanes (la κ-carraghénane, la i-carraghénane, la λ-carraghénane), la pectine, l'hydroxypropylcellulose (HPC), l'hydroxypropylmethylcellulose (HPMC), l'éthylcellulose (EC), le méthylcellulose (MC), l'hydroxypropyméthylcellulose acétosuccinate, la cellulose acétobutyrate, la cellulose acétophthalate, le glycogène, le β-glucane, l'amylopectine, l'amylose, les dextrines, la maltodextrine, l'isomaltose, le xylane, le pullulan, l'agar-agar, les mannanes, le fucoïdane, la gomme de xanthane, la gomme de guar, la gomme arabique, la gomme d'acacia, la gomme adragante, la gomme ghatti, la gomme mastic, la gomme de karaya, la gomme de Konjac, la gomme de fenugrec, la gomme d'acajou ou anacarde, la gomme tara, la gomme de caroube, la gomme de cassia, la gomme de benjoin, la gomme gaïac, le chitosan, la chitine, la lévane, la néoserine, l'acide hyaluronique, les hyaluronates, le sulfate de chondroïtine, le dermatane sulfate, le kératane sulfate, leurs dérivés et leurs mélanges.

A titre d'exemple, en ce qui concerne les alginates, peuvent être mentionnés l'alginate de calcium, l'alginate de sodium et l'alginate d'ammonium. Cette liste est non exhaustive.

Au sens de la présente invention, le terme « saccharide » regroupe les monosaccharides, les oligosaccharides et les polysaccharides.

A titre d'exemple, parmi les fibres solubles ou insolubles peuvent être mentionnées les fibres de Konjac, les fibres d'acacia, les fibres d'inuline, les fibres de chicorée ou encore les fibres d'arabinoxylane. Cette liste est non exhaustive.

A titre d'exemple, parmi les dextrines, peuvent être mentionnés les dextrines de pois, les dextrines de pomme de terre, les dextrines de maïs, leurs dérivés et leurs mélanges. Cette liste est non exhaustive.

A titre d'exemple, parmi les cyclodextrines, peuvent être mentionnés l'α-cyclodextrine, la β-cyclodextrine, la γ-cyclodextrine, leurs dérivés et leurs mélanges. Cette liste est non exhaustive.

A titre d'exemple, parmi les hyaluronates, peuvent être mentionnés l'acide hyaluronique, l'hyaluronate de sodium, leurs dérivés et leurs mélanges. Cette liste est non exhaustive.

A titre d'exemple, parmi les carraghénanes, peuvent être mentionnés la κ-carraghénane, la i-carraghénane, la λ-carraghénane, leurs dérivés et leurs mélanges. Cette liste est non exhaustive.

A titre d'exemple, parmi les amidons peuvent être mentionnés l'amidon de maïs, l'amidon de pomme de terre, l'amidon de riz, l'amidon de blé, l'amidon de sarrasin, l'amidon de châtaigne, l'amidon de patate douce ou encore l'amidon de manioc. Cette liste est non exhaustive.

Avantageusement, selon l'invention, ledit support naturel ou synthétique est un lipide choisi dans le groupe constitué des lipides suivants : les lécithines, les cires d'abeille, la cire de candelilla, la cire de son de riz, la cire de carnauba, les huiles animales ou végétales, les huiles animales ou végétales hydrogénées, les phospholipides, les céramides, les esters d'acide gras, leurs dérivés et leurs mélanges. Cette liste est non exhaustive.

Avantageusement, selon l'invention, ledit support naturel ou synthétique est un polymère synthétique choisi dans le groupe constitué des polymères synthétiques suivants : l'acétate de polyvinyle, le polyvinylpyrrolidone (PVP), l'acétate de polyvinylpyrrolidone-co-vinyle, l'acétate de polyethylène-co-vinyle, l'acétate co-méthacrylique d'acide polyvinyle, le polyéthylène oxide, le polylactide-co-glycolide, l'alcool polyvinylique, le polycarbophile, la polycaprolactone, le copolymère d'éthylène-vinyle, leurs dérivés et leurs mélanges. Cette liste est non exhaustive.

Par le terme « extrudat », il est entendu, au sens de la présente invention, une matière qui sort d'un appareillage, en particulier d'une extrudeuse, plus particulièrement de la filière d'une extrudeuse.

Par les termes « extrudat thermoformé », il est entendu, au sens de la présente invention, une matière qui sort d'un appareillage, en particulier qui sort d'une extrudeuse, dans lequel elle a subi une transformation par l'effet de la chaleur, éventuellement par l'effet combiné de la chaleur et de forces de cisaillement d'une vis sans fin. Une telle transformation par l'effet de la chaleur, éventuellement par l'effet combiné de la chaleur et de forces de cisaillement d'une vis sans fin, peut être obtenue avec la technique de l'extrusion à chaud (HME ou Hot Melt Extrusion).

Un extrudat thermoformé selon l'invention peut donc être obtenu selon la technique d'extrusion à chaud qui permet de réaliser la dispersion moléculaire d'un actif (substance active) au sein d'un support (par exemple au sein d'un polymère / d'une matrice polymérique) pour former des dispersions solides, l'actif et/ou le support mis en oeuvre subissant un changement d'état de la matière. L'actif et/ou le support passe donc d'un premier état de la matière à un second état de la matière, lequel second état de la matière impliquant que ledit actif et/ou ledit support se présente au moins partiellement sous forme amorphe. Cette dispersion solide et ce changement d'état de la matière sont possibles grâce à un apport de chaleur et éventuellement grâce à la contrainte appliquée par le mouvement des vis sans fin sur la matière dans un extrudeur. Préférentiellement, l'extrusion à chaud donne lieu, en sortie, à la formation d'un extrudat thermoformé, en particulier à la formation d'un extrudat thermoformé friable / cassant, sous forme d'un jonc qui peut alors notamment être pelletisé ou broyé.

En particulier, un extrudat thermoformé selon l'invention est un extrudat dans lequel au moins une dibenzo-α-pyrone naturelle ou synthétique comme substance active et/ou au moins un support naturel ou synthétique a/ont été fondu(s) pour donner lieu à une dispersion solide consistant en une structure vitreuse comprenant un mélange moléculaire de ladite au moins une dibenzo-α-pyrone naturelle ou synthétique comme substance active et dudit au moins un support naturel ou synthétique de telle sorte que ladite au moins une dibenzo-α-pyrone naturelle ou synthétique comme substance active est dispersée au sein dudit au moins un support naturel ou synthétique.

Par les termes « structure vitreuse », il est entendu, au sens de la présente invention, une structure comprenant / étant formée par un mélange moléculaire d'au moins deux composés / molécules, au moins l'un de ces deux composés / molécules se présentant au moins partiellement sous une forme non cristalline, en particulier se présentant au moins partiellement sous forme amorphe. En particulier, dans le cadre de la présente invention, une telle structure vitreuse est obtenue par un changement d'état de la matière comprenant le principe actif / la substance active (la dibenzo-α-pyrone) et/ou le support mis en oeuvre, lequel changement d'état de la matière est obtenu par extrusion à chaud, la matière étant soumise à un chauffage et subissant une fonte.

Par les termes « dispersion solide », il est entendu, au sens de la présente invention, un mélange / un système d'au moins deux composés / molécules, au moins l'un de ces deux composés / molécules se présentant au moins partiellement sous une forme non cristalline, en particulier se présentant au moins partiellement sous forme amorphe.

En particulier, une « dispersion solide » consiste en une dispersion moléculaire d'un principe actif / d'une substance active au moins partiellement sous forme amorphe au sein d'un support, par exemple au sein d'une matrice polymérique.

En particulier, un extrudat thermoformé selon l'invention, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, est un extrudat dans lequel le principe actif / la substance active (la dibenzo-α-pyrone) et/ou ledit au moins un support est/sont fondu(s).

En particulier, la composition selon l'invention, plus particulièrement la composition sous forme d'un extrudat thermoformé selon l'invention, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, est une dispersion solide dans laquelle ladite au moins une dibenzo-α-pyrone naturelle ou synthétique comme substance active est dispersée dans ledit au moins un support naturel ou synthétique.

Un extrudat thermoformé selon l'invention, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, est avantageusement obtenu par thermoformage à chaud, en particulier par thermoformage à chaud par la technique d'extrusion à chaud. Avantageusement, selon l'invention, le thermoformage à chaud concerne donc plus particulièrement la technique de l'extrusion à chaud.

Avantageusement, selon l'invention, l'extrusion à chaud fait que l'actif (la dibenzo-α-pyrone) et/ou le support naturel ou synthétique sont (de préférence simultanément) mélangés, chauffés, fondus, homogénéisés et extrudés. Un mélange intense et une agitation forcée notamment par les vis sans fin pendant le procédé d'extrusion à chaud cause la désagrégation / la distribution des particules d'actif dans le support naturel ou synthétique fondu donnant lieu à une dispersion solide. La matière (actif + support d'origine naturelle) se trouve finalement dans un « glassy state » ou « glassy structure » ou état vitreux et se comporte comme un solide friable / cassant, mais sans une structure totalement cristalline et ayant un faible arrangement.

Préférentiellement, il est donc prévu selon l'invention une composition obtenue par thermoformage à chaud sous forme d'un extrudat thermoformé, en particulier sous forme d'un extrudat thermoformé friable / cassant, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, obtenu par thermoformage à chaud, en particulier obtenu par extrusion à chaud, ladite composition comprenant au moins une dibenzo-α-pyrone naturelle ou synthétique comme substance active et au moins un support naturel ou synthétique.

Un extrudat thermoformé selon l'invention, en particulier un extrudat thermoformé friable / cassant selon l'invention, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, peut donc être obtenu selon la technique d'extrusion à chaud qui permet de réaliser la dispersion moléculaire d'un actif (substance active) au sein d'un support, en particulier au sein d'une matrice polymérique (au sein d'un polymère), pour former des dispersions solides. Cette dispersion solide est possible grâce à un apport de chaleur et éventuellement grâce à la contrainte appliquée par le mouvement des vis sans fin sur la matière dans un extrudeur. En final, généralement, l'extrusion à chaud donne lieu, en sortie, à la formation d'un extrudat thermoformé friable / cassant sous forme d'un jonc qui peut alors notamment être pelletisé ou broyé.

La technique d'extrusion à chaud peut être réalisée sans apport d'une phase liquide mais repose sur un apport de chaleur (chauffage) pour assurer une transformation de la matière (changement d'état de la matière) par thermoformage. La technique d'extrusion à chaud donne lieu à une transformation de la matière (changement d'état de la matière), en particulier à une structure vitreuse (« glassy structure ») obtenue sous l'action de la chaleur (chauffage), les particules constituant les poudres n'étant plus toutes présentes sous leur forme initiale (native) cristalline en fin de procédé d'extrusion à chaud mais ayant subis une transformation par thermoformage.

Selon l'invention, de préférence, ladite dibenzo-α-pyrone naturelle ou synthétique comme substance active comprend au moins une première phase amorphe et éventuellement une deuxième phase cristalline.

Par les termes « comprend au moins une première phase amorphe et éventuellement une deuxième phase cristalline », il est entendu, au sens de la présente invention, que ladite dibenzo-α-pyrone naturelle ou synthétique comme substance active peut soit comprendre 100% en masse d'une phase amorphe, soit qu'elle peut comprendre simultanément une première phase amorphe et une deuxième phase cristalline, la somme des pourcentages en masse des première et deuxième phases étant dans ce cas égale à 100. En d'autres termes, la composition selon l'invention peut comprendre ladite dibenzo-α-pyrone naturelle ou synthétique comme substance active (1) totalement sous forme amorphe ou (2) partiellement sous forme (phase) amorphe et partiellement sous forme (phase) cristalline.

Notons qu'une phase est dite amorphe lorsque les atomes la constituant ne respectent aucun ordre à moyenne et grande distance, ce qui la distingue d'une phase dite cristalline.

Dans le cadre de la présente invention, une analyse par calorimétrie différentielle à balayage (DSC) qui est bien connue de l'homme de métier peut être utilisée afin de mesurer la différence d'échange de chaleur entre une référence et un produit étudié. Cette technique permet de déterminer les températures de fusion, les températures de cristallisation ou les températures de transitions de phase mais aussi les enthalpies de réaction. En particulier, une telle analyse permet d'obtenir des courbes où la présence d'un pic endothermique (absorption de la chaleur) montre la fusion de l'échantillon analysé. Un pic de fusion indique la présence d'une forme cristalline dans l'échantillon. Au contraire, lorsque le pic de fusion n'est pas présent, le matériel est à l'état amorphe.

La composition selon l'invention se présente donc de préférence sous forme d'un extrudat thermoformé, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, dans lequel ladite dibenzo-α-pyrone naturelle ou synthétique comme substance active comprend au moins une première phase amorphe et éventuellement une deuxième phase cristalline, laquelle ou lesquelles phases(s) est/sont dispersée(s) au sein d'au moins un support naturel ou synthétique.

Il a été déterminé, dans le cadre de la présente invention, qu'une telle composition sous forme d'un extrudat thermoformé, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, comprenant au moins une dibenzo-α-pyrone naturelle ou synthétique comme substance active et au moins support naturel ou synthétique, présente une solubilité et/ou une dispersion en phase aqueuse (milieu aqueux) nettement supérieure de ladite au moins une dibenzo-α-pyrone naturelle ou synthétique comme substance active. Par ailleurs, il a été montré qu'une telle composition selon l'invention présente une biodisponibilité / bioaccessibilité significativement augmentée de ladite au moins une dibenzo-α-pyrone naturelle ou synthétique comme substance active par rapport aux biodisponibilités / bioaccessibilités observées pour les compositions actuelles comprenant au moins une dibenzo-α-pyrone naturelle ou synthétique.

Selon l'invention, le principe actif / la substance active, c'est-à-dire ladite dibenzo-α-pyrone naturelle ou synthétique est dispersée / répartie / distribuée de manière homogène au sein d'au moins un support naturel ou synthétique, le principe actif et/ou ledit au moins un support naturel ou synthétique étant fondu(s) lors du procédé de fabrication par thermoformage mis en oeuvre selon l'invention et décrit plus loin.

En outre, une composition selon l'invention peut être conservée plusieurs mois sans que ses propriétés ne soient altérées. En particulier, il a été mis en évidence qu'une composition selon l'invention conserve ses propriétés en termes de solubilité et/ou de dispersion en phase aqueuse (milieu aqueux) de ladite au moins une dibenzo-α-pyrone naturelle ou synthétique comme substance active et en termes de taux de relargage de ce composé au cours du temps au départ d'une composition / formulation selon l'invention, ceci en particulier en phase aqueuse.

Avantageusement, selon l'invention, ledit extrudat thermoformé, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, comprend un mélange thermoformé de ladite au moins une dibenzo-α-pyrone naturelle ou synthétique comme substance active et d'au moins un support naturel ou synthétique.

Alternativement, selon l'invention, ledit extrudat thermoformé, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, est constitué d'un mélange thermoformé de ladite au moins une dibenzo-α-pyrone naturelle ou synthétique comme substance active et d'au moins un support naturel ou synthétique.

Il est donc prévu selon l'invention un extrudat thermoformé, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, obtenu par thermoformage à chaud, en particulier obtenu par extrusion à chaud, ledit extrudat thermoformé comprenant un mélange thermoformé d'au moins une dibenzo-α-pyrone naturelle ou synthétique comme substance active et d'au moins un support naturel ou synthétique.

Par les termes « mélange thermoformé », il est entendu, au sens de la présente invention, un mélange qui sort d'un appareillage, en particulier qui sort d'une extrudeuse, dans lequel il a subi une transformation par l'effet de la chaleur, éventuellement par l'effet combiné de la chaleur et de forces de cisaillement d'une vis sans fin. Une telle transformation par l'effet de la chaleur, éventuellement par l'effet combiné de la chaleur et de forces de cisaillement d'une vis sans fin, peut être obtenue avec la technique de l'extrusion à chaud (HME).

En particulier, un mélange thermoformé selon l'invention est un mélange dans lequel le principe actif / la substance active (la dibenzo-α-pyrone) et/ou ledit au moins un support naturel ou synthétique est/sont fondu(s) / a été/ont été fondu(s).

Un mélange thermoformé selon l'invention peut donc être obtenu selon la technique d'extrusion à chaud qui permet de réaliser la dispersion moléculaire d'un actif (substance active) au sein d'un support naturel ou synthétique, par exemple au sein d'une matrice polymérique (au sein d'un polymère) pour former des dispersions solides, l'actif et/ou le support naturel ou synthétique mis en oeuvre subissant un changement d'état de la matière. L'actif et/ou le support naturel ou synthétique passe donc d'un premier état de la matière à un second état de la matière, lequel second état de la matière impliquant que ledit actif et/ou ledit support naturel ou synthétique se présente au moins partiellement sous forme amorphe.

Préférentiellement, selon l'invention, ladite au moins une dibenzo-α-pyrone naturelle ou synthétique comme substance active comprend une première phase amorphe.

Avantageusement, selon l'invention, ladite au moins une dibenzo-α-pyrone naturelle ou synthétique comme substance active comprend majoritairement au moins une première phase amorphe.

De préférence, selon l'invention, ladite au moins une dibenzo-α-pyrone naturelle ou synthétique comme substance active comprend entre 51 et 100% en masse d'une phase amorphe et entre 0 et 49% d'une phase cristalline.

Par les termes « majoritairement au moins une première phase amorphe », il est donc entendu, au sens de la présente invention, que ladite au moins une dibenzo-α-pyrone naturelle ou synthétique comme substance active comprend entre 50 et 100% en masse d'une phase amorphe et entre 0 et 50% d'une phase cristalline, plus particulièrement que ladite au moins une dibenzo-α-pyrone naturelle ou synthétique comme substance active comprend entre 51 et 100% en masse d'une phase amorphe et entre 0 et 49% d'une phase cristalline.

De préférence, selon l'invention, ladite dibenzo-α-pyrone naturelle ou synthétique comme substance active est choisie dans le groupe constitué des composés suivants : l'urolithine A, l'isourolithine A, l'urolithine B, l'urolithine C, l'urolithine D, l'urolithine E, l'urolithine M-5, l'urolithine M-6, l'urolithine M-7, l'isourolithine B, la 8-O-methylurolithine A, la 8,9-di-O-methylurolithine C, et la 8,9-di-O-methylurolithine D. En ce sens, avantageusement, ladite dibenzo-α-pyrone naturelle ou synthétique comme substance active est un composé dont la structure chimique de base est la suivante (Formule générale (II)) : 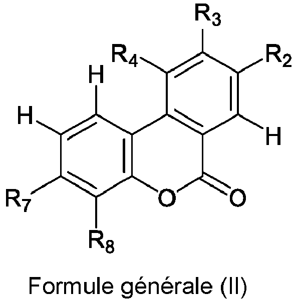 dans laquelle,
R₂ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₃ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₄ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₇ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₈ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté ; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4.

Avantageusement, la composition selon l'invention comprend en outre au moins un agent plastifiant. L'addition d'un agent plastifiant dans une composition selon l'invention permet d'obtenir une composition selon l'invention au travers d'un procédé de fabrication où des températures inférieures aux points de fusion de ladite au moins une dibenzo-α-pyrone naturelle ou synthétique comme substance active et du support naturel ou synthétique peuvent être utilisées afin de garantir tout de même une fonte de ces deux composés et la dispersion / répartition / distribution de ladite au moins une dibenzo-α-pyrone naturelle ou synthétique comme substance active au sein du support naturel ou synthétique.

Préférentiellement, selon l'invention, ledit agent plastifiant est choisi dans le groupe constitué des polyols, des lipides, des lécithines, des esters de sucrose, de l'eau, du citrate de triéthyle, du polyéthylène glycol, du glycérol, du sébate de dibutyle, du stéarate de butyle, du monostéarate de glycérol, du sodium dodécylsulphate, du phtalate diéthyle, de leurs dérivés et de leurs mélanges. Cette liste est non exhaustive.

Selon l'invention, les agents plastifiants préférés sont le glycérol, l'eau, le polyéthylène glycol, le citrate de triéthyle, les lécithines, les polyols et les esters de sucrose
Avantageusement, selon l'invention, ladite dibenzo-α-pyrone naturelle ou synthétique comme substance active est présente à raison de 1% à 80% en poids par rapport au poids total de la composition, de préférence à raison de 10% à 60% en poids par rapport au poids total de la composition, de préférence à raison de 15% à 50% en poids par rapport au poids total de la composition, préférentiellement à raison de 20% à 40% en poids par rapport au poids total de la composition.

Préférentiellement, selon l'invention, ladite dibenzo-α-pyrone naturelle ou synthétique comme substance active est présente à raison de 20% en poids par rapport au poids total de la composition, ou à raison de 25% en poids par rapport au poids total de la composition, ou à raison de 30% en poids par rapport au poids total de la composition, ou à raison de 35% en poids par rapport au poids total de la composition, ou à raison de 40% en poids par rapport au poids total de la composition.

De préférence, selon l'invention, ledit au moins un support naturel ou synthétique est présent à raison de 5% à 90% en poids par rapport au poids total de la composition, de préférence à raison de 15% à 85% en poids par rapport au poids total de la composition, préférentiellement à raison de 20% à 80% en poids par rapport au poids total de la composition, plus préférentiellement à raison de 50% à 75% en poids par rapport au poids total de la composition.

Avantageusement, selon l'invention, ledit au moins un support naturel ou synthétique est présent à raison de 5% en poids par rapport au poids total de la composition, ou à raison de 10% en poids par rapport au poids total de la composition , ou à raison de 15% en poids par rapport au poids total de la composition, ou à raison de 20% en poids par rapport au poids total de la composition, ou à raison de 25% en poids par rapport au poids total de la composition, ou à raison de 30% en poids par rapport au poids total de la composition, ou à raison de 35% en poids par rapport au poids total de la composition, ou à raison de 40% en poids par rapport au poids total de la composition, ou à raison de 45% en poids par rapport au poids total de la composition, ou à raison de 50% en poids par rapport au poids total de la composition, ou à raison de 55% en poids par rapport au poids total de la composition, ou à raison de 60% en poids par rapport au poids total de la composition, ou à raison de 65% en poids par rapport au poids total de la composition, ou à raison de 70% en poids par rapport au poids total de la composition, ou à raison de 75% en poids par rapport au poids total de la composition.

Lorsqu'il s'agit d'un mélange de deux supports selon l'invention, le ratio entre ces deux supports est préférentiellement de l'ordre de 10:90 ou de l'ordre de 20:80 ou de l'ordre de 30:70 ou de l'ordre de 40:60 ou de l'ordre de 50:50.

Avantageusement, selon l'invention, ledit au moins un agent plastifiant est présent à raison de 1% à 30% en poids par rapport au poids total de la composition, de préférence à raison de 10% à 25% en poids par rapport au poids total de la composition, préférentiellement à raison de 15% à 20% en poids par rapport au poids total de la composition.

Avantageusement, selon l'invention, ledit au moins un agent plastifiant est présent à raison de 5% en poids par rapport au poids total de la composition, ou à raison de 10% en poids par rapport au poids total de la composition, à raison de 15% en poids par rapport au poids total de la composition, à raison de 20% en poids par rapport au poids total de la composition, à raison de 25% en poids par rapport au poids total de la composition, à raison de 30% en poids par rapport au poids total de la composition.

De préférence, la composition selon l'invention comprend en outre au moins un additif choisi dans le groupe constitué des agents lubrifiants, des agents surfactants, des agents antioxydants, des agents chélatants, de leurs dérivés et de leurs mélanges.

A titre d'exemple, peuvent être utilisés, seuls ou en mélange, en tant qu'agents lubrifiants dans une composition selon l'invention les composés suivants : le dibéhénate de glycérol, le talc, la silice, l'acide stéarique, l'acide borique, les cires, l'oléate de sodium, l'acétate de sodium, le stéarate de magnésium, le stéarate de calcium, le stéarate de sodium, le benzoate de sodium, le laurylsulfate de sodium, le distéarate de glycérol, le palmitostéarate de glycérol, la cellulose microcristalline ou encore les polyoxyl-8-glycérides.

A titre d'exemple, peuvent être utilisés, seuls ou en mélange, en tant qu'agents surfactants dans une composition selon l'invention les composés suivants : le Pluronic^{®}, le Span^{®}, le Cremophor^{®}, les polysorbates (Tween^{®}, ...), la vitamine E TPGS et le ducosate de sodium.

A titre d'exemple, peuvent être utilisés, seuls ou en mélange, en tant qu'agents antioxydants et/ou agents chélatants dans une composition selon l'invention les composés suivants : l'hydroxytoluène butylé, l'hydroxyanisiole butylé, l'EDTA, l'acide citrique, l'acide ascorbique et la vitamine E.

Avantageusement, la composition selon l'invention comprend en outre au moins un composé additionnel de type polyphénol choisi dans le groupe constitué des acides phénoliques, des stilbènes, des alcools phénoliques, des lignanes, des flavonoïdes, de leurs dérivés et de leurs mélanges. En particulier, les formes glycosylée et aglycone des polyphénols sont envisagées en tant que principe actif additionnel selon la présente invention. Plus particulièrement, au sens de la présente invention, le terme « polyphénol » désigne tant les polyphénols d'origine naturelle que les polyphénols de synthèse mais aussi tous les dérivés de polyphénols.

A titre d'exemple, au sens de la présente invention, peuvent être cités en tant qu'acides phénoliques, les dérivés de l'acide hydroxybenzoïque (acide gallique, acide tannique, ...) et les dérivés de l'acide hydroxycinamique (curcumine, acide coumarique, acide caféique, acide férulique, ...).

A titre d'exemple, au sens de la présente invention, peuvent être cités en tant que stilbènes, le resvératrol, le sirtinol, le picéatannol ou encore la polydatine.

A titre d'exemple, au sens de la présente invention, peuvent être cités en tant que flavonoïdes, les flavanoles (quercétine, myricétine, kaempférol, isorhamnétine, morine, rutine, tiliroside, trihydroxyéthylrutine, fisétine, ...), les flavones (apigénine, lutéoline, baicaléine, chrysine, diosmine, nobilétine, tangérétine, wogonine, aminogénistéine, ...), les flavanones (bavachine, 8-isopenténylnaringénine, isoxanthohumole, naringénine, eriodictyole, hespérétine, silybine, taxifoline, ...), les isoflavones (génistéine, daidzéine, daidzine, formonétine, génistine, néobavaisoflavone, puéranine, ...), les antocianidines (cianidine, pelargonidine, delphinidine, pétunidine, malvidine, ...) et les flavanols (cathéchines, gallocatéchine, épigallocatéchinegallate, ...).

Selon l'invention, ledit au moins un principe actif additionnel de type polyphénol constitue un inhibiteur/modulateur des pompes à efflux dont la P-gp.

Préférentiellement, la composition selon l'invention comprend en outre au moins un inhibiteur et/ou un modulateur de l'activité de la P-gp.

De préférence, la composition selon l'invention est conditionnée sous forme de pellets, de flakes, de granulés, de poudres, de comprimés effervescents ou non, de solutions injectables ou non, de suspensions, de gels, de pommades ou encore sous toute autre forme adéquate permettant une administration à un animal ou à un être humain.

D'autres formes de réalisation d'une composition suivant l'invention sont indiquées dans les revendications annexées.

L'invention a aussi pour objet un procédé de fabrication, en particulier un procédé de fabrication par thermoformage, d'une composition sous forme d'un extrudat thermoformé selon l'invention, caractérisé en ce qu'il comprend les étapes suivantes :
a) une étape d'amenée simultanée ou différée dans le temps d'au moins une dibenzo-α-pyrone naturelle ou synthétique comme substance active et d'au moins un support naturel ou synthétique, pour alimenter un extrudeur,
b) une étape de mélange, dans ledit extrudeur, de ladite au moins une dibenzo-α-pyrone naturelle ou synthétique comme substance active et dudit au moins un support naturel ou synthétique, pour former un mélange, et
c) une étape d'extrusion à chaud dudit mélange obtenu à l'étape b) dans ledit extrudeur pour obtenir un extrudat thermoformé, en particulier pour obtenir un extrudat thermoformé consistant en une dispersion solide, plus particulièrement pour obtenir un extrudat thermoformé consistant en une dispersion solide consistant en une structure vitreuse comprenant un mélange moléculaire de ladite au moins une dibenzo-α-pyrone naturelle ou synthétique comme substance active et dudit au moins un support naturel ou synthétique.

Préférentiellement, selon l'invention, les étapes a) et b) ont lieu simultanément.

Préférentiellement, selon l'invention, les étapes a), b) et c) ont lieu simultanément.

Un tel procédé selon l'invention donne lieu à une composition sous forme d'un extrudat thermoformé, en particulier à une composition sous forme d'un extrudat thermoformé friable / cassant, c'est-à-dire obtenu par thermoformage et plus particulièrement par extrusion à chaud, dans lequel ladite au moins une dibenzo-α-pyrone naturelle ou synthétique comme substance active comprend préférentiellement au moins une première phase amorphe et éventuellement une deuxième phase cristalline dispersée(s) au sein dudit au moins un support naturel ou synthétique.

Cette composition suivant l'invention présente une solubilité et/ou une dispersion en phase aqueuse (milieu aqueux) nettement supérieure de ladite au moins une dibenzo-α-pyrone naturelle ou synthétique comme substance active et simultanément une biodisponibilité / bioaccessibilité significativement augmentée de ladite au moins une dibenzo-α-pyrone naturelle ou synthétique comme substance active par rapport aux solubilités et/ou aux dispersions et aux biodisponibilités / bioaccessibilités de ces composés pour les compositions actuelles. Il a été montré que la composition selon l'invention se présente sous forme d'un extrudat thermoformé, en particulier sous forme d'un extrudat thermoformé friable / cassant, dans lequel ladite au moins une dibenzo-α-pyrone naturelle ou synthétique comme substance active (principe actif) comprenant préférentiellement au moins une première phase amorphe et éventuellement une deuxième phase cristalline est dispersée au sein dudit au moins un support naturel ou synthétique.

Il a également été montré, dans le cadre de la présente invention, que ladite au moins une dibenzo-α-pyrone naturelle ou synthétique comme substance active n'est pas dégradée ou du moins qu'elle n'est dégradée que dans une moindre mesure même lors du procédé de fabrication par thermoformage de la composition sous forme d'un extrudat thermoformé, en particulier sous forme d'un extrudat thermoformé friable / cassant, qui implique pourtant la soumission de ladite au moins une dibenzo-α-pyrone naturelle ou synthétique comme substance active à de hautes températures (HME). Par ailleurs, il a également été mis en avant que ladite au moins une dibenzo-α-pyrone naturelle ou synthétique comme substance active dans une composition sous forme d'un extrudat thermoformé suivant l'invention, en particulier sous forme d'un extrudat thermoformé friable / cassant suivant l'invention, y est réparti (distribué) de façon homogène.

Plus particulièrement, l'extrusion à chaud (Hot Melt Extrusion - HME) réalisée selon le procédé de fabrication par thermoformage suivant l'invention donne lieu à une fonte du principe actif / de la substance active (la dibenzo-α-pyrone naturelle ou synthétique) et/ou dudit au moins un support naturel ou synthétique à une température supérieure ou égale à leur point de fusion.

Toutefois, selon certains modes de réalisation d'une composition selon l'invention, cette fonte du principe actif / de la substance active et/ou du support naturel ou synthétique peut avoir lieu à une température inférieure à leur point de fusion. Ceci est par exemple le cas si la composition selon l'invention comprend un agent plastifiant ou si le principe actif / la substance active lui-même présente des propriétés plastifiantes. Une telle fonte du principe actif / de la substance active et/ou du support naturel ou synthétique donne lieu à une dispersion solide dans laquelle le principe actif / la substance active (ladite dibenzo-α-pyrone naturelle ou synthétique) comprenant préférentiellement au moins une première phase amorphe et éventuellement une deuxième phase cristalline est dispersé / réparti / distribué de façon homogène au sein dudit au moins un support naturel ou synthétique.

Avantageusement, selon le procédé suivant l'invention, l'étape c) d'extrusion à chaud est une étape selon laquelle ladite dibenzo-α-pyrone naturelle ou synthétique et/ou ledit au moins un support naturel ou synthétique subisse/subissent un changement d'état de la matière, ladite au moins une dibenzo-α-pyrone naturelle ou synthétique comme substance active et/ou ledit au moins un support naturel ou synthétique passant d'un premier état de la matière à un second état de la matière, lequel second état de la matière impliquant en particulier que ladite au moins une dibenzo-α-pyrone naturelle ou synthétique comme substance active et/ou ledit au moins un support naturel ou synthétique se présente/présentent au moins partiellement sous forme amorphe.

Avantageusement, le procédé suivant l'invention comprend une étape préalable de pré-mélange de ladite au moins une dibenzo-α-pyrone naturelle ou synthétique comme substance active et dudit au moins un support naturel ou synthétique, de telle sorte à former un pré-mélange destiné à alimenter l'extrudeur.

De façon préférée, selon le procédé suivant l'invention, ladite étape d'extrusion à chaud est réalisée à une température d'extrusion ou température de thermoformage comprise entre 20 et 300°C.

Avantageusement, selon le procédé suivant l'invention, ladite étape d'extrusion à chaud est réalisée à une vitesse de rotation d'une vis d'extrusion comprise entre 20 et 900 tours/min, de préférence comprise entre 50 et 300 tours/min, de préférence comprise entre 100 et 250 tours/min, préférentiellement égale à 250 tours/min, préférentiellement égale à 200 tours/min, préférentiellement égale à 100 tours/min.

Préférentiellement, le procédé selon l'invention comprend une étape additionnelle de refroidissement en sortie de l'extrudeur.

Avantageusement, le procédé selon l'invention comprend une étape additionnelle de traitement de l'extrudat thermoformé, en particulier de l'extrudat thermoformé friable / cassant, en sortie de l'extrudeur, par exemple une découpe au niveau d'un pelletiseur et/ou un broyage et/ou un concassage et/ou une réduction en poudre (sous forme de poudre) dudit extrudat thermoformé, en particulier dudit extrudat thermoformé friable / cassant.

D'autres formes de réalisation du procédé suivant l'invention sont indiquées dans les revendications annexées.

La présente invention porte aussi sur une composition sous forme d'un extrudat thermoformé, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, obtenu suivant le procédé selon l'invention.

La présente invention porte également sur une composition sous forme d'un extrudat thermoformé, en particulier sous forme d'un extrudat thermoformé friable / cassant, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, obtenu suivant le procédé selon l'invention, ladite composition comprenant au moins une dibenzo-α-pyrone naturelle ou synthétique comme substance active et au moins un support naturel ou synthétique, ladite au moins une dibenzo-α-pyrone naturelle ou synthétique comme substance active comprenant préférentiellement au moins une première phase amorphe et éventuellement une deuxième phase cristalline.

En d'autres termes, la présente invention porte également sur une composition sous forme d'un extrudat thermoformé, en particulier sous forme d'un extrudat thermoformé friable / cassant, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, obtenu par extrusion à chaud (HME - Hot Melt Extrusion), ladite composition comprenant au moins une dibenzo-α-pyrone naturelle ou synthétique comme substance active et au moins un support naturel ou synthétique, ladite au moins une dibenzo-α-pyrone naturelle ou synthétique comme substance active comprenant préférentiellement au moins une première phase amorphe et éventuellement une deuxième phase cristalline.

La présente invention porte également sur une utilisation d'une composition suivant l'invention en tant que complément alimentaire et/ou en tant que produit cosmétique et/ou en tant que médicament à usage humain ou vétérinaire et/ou en tant que composé administré via un dispositif médical, par exemple via un spray ou via un patch.

En particulier, la présente invention porte sur une composition pour utilisation dans le traitement préventif et/ou curatif, chez l'être humain et/ou chez l'animal, de pathologies en lien avec le système musculaire et neuromusculaire, de pathologies en lien avec la colonne vertébrale et les articulations, de pathologies en lien avec le système cardiovasculaire (ischémie, désordres métaboliques, dyslipidémies...), de pathologies en lien avec le système hormonal, de pathologies en lien avec le système gastrointestinal (nausées, vomissements, diarrhées, inflammations digestives, ...), de pathologies liées au système rénal (néphropathies, lésions rénales aigues, ... ), de pathologies en lien avec le vieillissement prématuré des cellules, antioxydant, de pathologies en lien avec le système endocrinien (hyperglycémie, ...), de pathologies en lien avec le système immunitaire (maladies inflammatoires, myopathies, arthrite rhumatoïde, sclérose en plaque, ostéoarthrite, fibromyalgie,...), de pathologies en lien avec le système nerveux central (douleur, migraine, épilepsie, maladie de Parkinson, maladie d'Alzheimer, anxiété, dépression,...), des troubles du sommeil, de maladies de la peau, de maladies dues à la présence de microorganismes, des cancers (anti-tumoral, ...), des maladies oculaires (glaucoma,...), des maladies du système respiratoire (asthme,...) et dans le traitement préventif et/ou curatif du diabète.

Plus particulièrement, la présente invention porte sur une composition pour utilisation dans le traitement préventif et/ou curatif, chez l'être humain et/ou chez l'animal, des maladies liées aux désordres neuropsychiatriques, de la douleur, des maladies neurodégénératives, des maladies inflammatoires, des désordres métaboliques et rénaux, des désordres musculo-squelettiques, des désordres hormonaux, des cancers, des maladies cardiovasculaires, de l'ostéoporose, de l'endométriose, des dermatites et des maladies de la peau ou encore du côlon irritable (IBS).

Plus particulièrement, la présente invention porte sur une composition utilisée, chez l'être humain et/ou chez l'animal, en tant qu'antioxydant, qu'anti-inflammatoire, qu'anticonvulsif, qu'antiémétique, qu'anxiolytique, qu'hypnotique ou encore en tant qu'antipsychotique.

Une composition selon l'invention présente préférentiellement des propriétés anabolisantes, analgésiques, relaxantes, anesthésiques, antithrombotiques, antivirales, antifongiques et antibactériennes, antimalariques, insecticides, immunomodulatrices, de modulateur hormonal, de modulateur oestrogénique, androgéniques, spermatogéniques, antimutagéniques, antiépileptiques, anti-inflammatoires, neuroprotectrices, anticonvulsivantes, anxyolitiques, antidepressions, antipsychotiques, antithrombotiques, antitumorales, antidiabétiques et/ou immunorégulatrices.

D'autres formes d'utilisation d'une composition suivant l'invention sont indiquées dans les revendications annexées.

### Exemples

### Exemple 1: procédé de fabrication par thermoformage de compositions selon l'invention sous forme d'extrudats thermoformés

Des compositions thermoformées selon l'invention comprenant au moins une dibenzo-α-pyrone, telles que celles faisant l'objet de l'exemple 2 ci-dessous, ont été obtenues selon le procédé suivant faisant également l'objet de la présente invention :
a) une étape de pré-mélange d'au moins une dibenzo-α-pyrone à l'état natif sous forme de poudre et d'au moins un support naturel ou synthétique ;
b) une étape d'amenée dudit pré-mélange formé à l'étape a) pour alimenter un extrudeur de type Process 11 Hygienic de Thermo-Fischer^{®} ;
c) une étape de mélange, dans ledit extrudeur, dudit pré-mélange pour obtenir un mélange ;
d) une étape de thermoformage par extrusion à chaud dudit mélange obtenu à l'étape c) dans ledit extrudeur pour obtenir un extrudat thermoformé, en particulier un extrudat thermoformé friable / cassant, l'étape d'extrusion à chaud étant réalisée à une vitesse de rotation d'une vis d'extrusion de 100 tours/minute et à une température comprise entre 20 et 160°C ;
e) une étape de refroidissement en sortie de l'extrudeur dudit extrudat thermoformé, en particulier dudit extrudat thermoformé friable / cassant, obtenu à l'étape d) ; et
f) une étape de découpe / broyage, au niveau d'un broyeur, de l'extrudat thermoformé refroidi obtenu à l'étape e), en particulier de l'extrudat thermoformé friable / cassant refroidi obtenu à l'étape e), de telle sorte à obtenir une poudre homogène, c'est-à-dire un extrudat thermoformé conditionné en poudre (sous forme de poudre).

La température de thermoformage à laquelle est réalisée l'étape d'extrusion à chaud est déterminée par le type de constituants mis en oeuvre, en particulier selon le type de support et/ou d'agent plastifiant mis en oeuvre, ce que l'homme de métier est à même de déterminer. Par ailleurs, un homme de métier, notamment selon le type d'extrudeur employé et conformément au principe général de l'extrusion à chaud (HME), est à même de définir d'éventuels paliers de températures en différentes zones le long de la ou des vis d'extrusion de telle sorte qu'ait lieu une augmentation progressive de la température au sein de la matière transportée par la ou des vis d'extrusion, ceci dans un sens d'avancement de la matière au sein de l'extrudeur. Typiquement, entre zones définies le long de la ou des vis d'extrusion, des différences de températures de l'ordre de 0 à 40°C sont observées. Par exemple, dans le cadre de la présente invention, les compositions testées ci-après ont été obtenues dans un extrudeur de type Process 11 Hygienic de Thermo-Fischer^{®} présentant 8 zones de températures qui sont les suivantes dans un sens d'avancée de la matière évoluant à une vitesse de 100 tours/minute : 30°C - 60°C - 100°C - 120°C - 140°C - 150°C - 160°C et 160°C.

### Exemple 2 : tests de dispersion et de solubilité de compositions thermoformées selon l'invention

Des compositions thermoformées, obtenues selon le procédé de fabrication décrit à l'exemple 1, ont été testées en termes de dispersion et de solubilité de l'urolithine A ou de l'urolithine B. Cette dispersion et cette solubilité ont été mesurées au cours du temps au départ d'extrudats thermoformés obtenus selon l'invention. Comme indiqué ci-dessus, les extrudats thermoformés se présentent sous la forme d'une poudre homogène (broyat) dans laquelle l'urolithine A ou l'urolithine B comprend préférentiellement au moins une phase amorphe.

Le test de dispersion et de solubilité a été réalisé avec un appareil à dissolution à palettes au départ d'environ 800 mg d'extrudat thermoformé (environ 160 mg d'urolithine A ou d'urolithine B), à une température de 37°C sous agitation à 50 tours/minute dans 900 ml d'un milieu de dissolution HCl 0,1N. Ce test de dispersion et de solubilité a été mené selon les recommandations de la pharmacopée Ph.Eur.9.0 (Recommendations on Dissolution Testing). A des temps déterminés (après 15 min, après 30 min et après 1h), un échantillon de 1 ml est prélevé pour réaliser le test de dispersion et de solubilité.

Afin de réaliser le test de dispersion et de solubilité, l'échantillon est dilué dans un solvant approprié (phase mobile : mélange méthanol (70%) - isopropanol (30%) puis filtré au travers d'un filtre (PET, taille des pores de 0,45 µm, Macherey Nagel) avant analyse HPLC (colonne Nucleodur 100-5 EC C18 125/4 (Macherey-Nagel) ; phase mobile : 70% méthanol et 30% isopropanol ; débit : 0,6 ml/min ; loop : 10 µl, t° = 25°C ; 210 nm).

Les compositions thermoformées selon l'invention reprises aux Tableaux 1 et 2 ont été formulées suivant le procédé de l'invention et testées en termes de dispersion et de solubilité au cours du temps selon le principe indiqué ci-dessus. De l'urolithine A ou de l'urolithine B sous forme native cristalline et sous forme de poudre (urolithine A native ou urolithine B native) a été utilisé en tant que contrôle. Les quantités mentionnées aux Tableaux 1 et 2 sont des pourcentages en poids des composés mis en oeuvre (soumis au procédé selon l'invention) par rapport au poids total de la composition.

**Tableau 1**

| | Urolithine A (1) | Protéine (2) | Glycérol (3) |
|---|---|---|---|
| Compo 1 | 20 | 70 | 10 |

| | | | |
|---|---|---|---|
| (1) Urolithine A (Pincredit Bio-Tech) (2) Peptides de collagène (Peptan B) (Rousselot) (3) Glycérol (Brenntag) | | | |

**Tableau 2**

| | Urolithine B (1) | Protéine (2) | Glycérol (3) |
|---|---|---|---|
| Compo 1 | 20 | 70 | 10 |

| | | | |
|---|---|---|---|
| (1) Urolithine B (Cimasci) (2) Peptides de collagène (Peptan B) (Rousselot) (3) Glycérol (Brenntag) | | | |

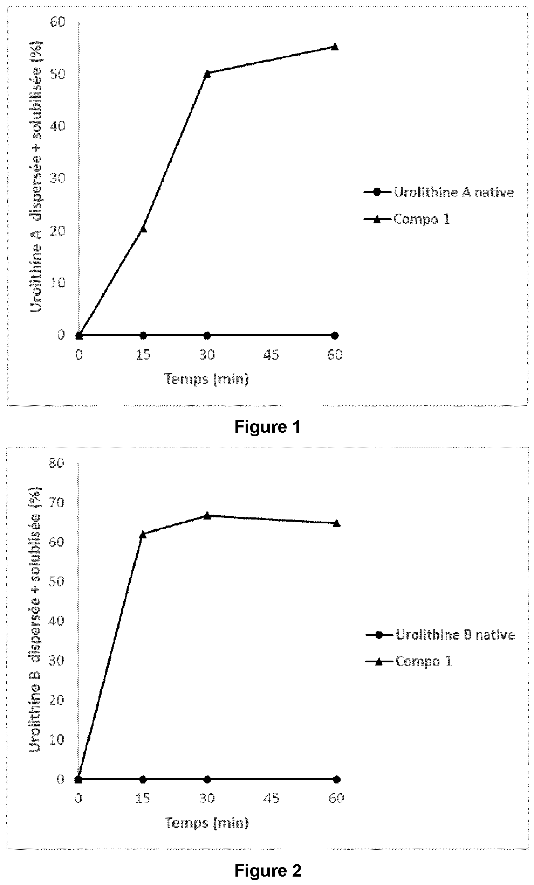

Les résultats des tests de dispersion et de solubilité obtenus sont présentés aux Figures 1 (urolithine A) et 2 (urolithine B). Comme on peut le constater, la dispersion + la solubilité mesurées pour l'urolithine A ou l'urolithine B au départ de compositions sous forme d'extrudats thermoformés suivant l'invention (Compo1) est nettement supérieure par rapport au contrôle (urolithine A native ou urolithine B native).

La présente invention a été décrite en relation avec des modes de réalisations spécifiques, qui ont une valeur purement illustrative et ne doivent pas être considérés comme limitatifs. D'une manière générale, il apparaîtra évident pour l'homme du métier que la présente invention n'est pas limitée aux exemples illustrés et/ou décrits ci-dessus.

L'usage des verbes « comprendre », « inclure », « comporter », ou toute autre variante, ainsi que leurs conjugaisons, ne peut en aucune façon exclure la présence d'éléments autres que ceux mentionnés.

L'usage de l'article indéfini « un », « une », ou de l'article défini « le », « la » ou « l' », pour introduire un élément n'exclut pas la présence d'une pluralité de ces éléments.

## Revendications

1. Composition sous forme d'un extrudat thermoformé, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, comprenant au moins une dibenzo-α-pyrone naturelle ou synthétique comme substance active et au moins un support naturel ou synthétique.

2. Composition selon la revendication 1, **caractérisée en ce que** ladite au moins une dibenzo-α-pyrone naturelle ou synthétique est un composé dont la structure chimique de base est la suivante (Formule générale (I)) : 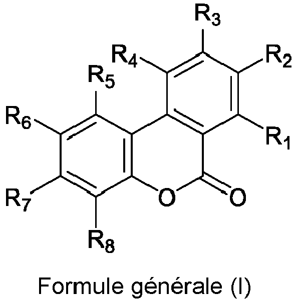 dans laquelle,
R₁ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₂ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₃ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₄ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₅ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₆ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₇ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₈ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4.

3. Composition selon la revendication 1 ou 2, **caractérisée en ce que** ledit support naturel ou synthétique est choisi dans le groupe constitué des acides aminés, des peptides et des polypeptides naturels ou synthétiques, des protéines naturelles ou synthétiques, des saccharides naturels ou synthétiques, des lipides naturels ou synthétiques, des polymères synthétiques, de l'urée, de leurs dérivés et de leurs mélanges.

4. Composition selon l'une quelconque des revendications précédentes, **caractérisée** en ce ladite dibenzo-α-pyrone naturelle ou synthétique est un composé choisi parmi l'urolithine A, l'isourolithine A, l'urolithine B, l'urolithine C, l'urolithine D, l'urolithine E, l'urolithine M-5, l'urolithine M-6, l'urolithine M-7, l'isourolithine B, l'urolithine A glucuronide, l'urolithine B glucuronide, la 8-Omethylurolithine A, la 8,9-di-O-methylurolithine C, et la 8,9-di-O-methylurolithine D.

5. Composition selon la revendication 4, **caractérisée en ce que** ladite au moins une dibenzo-α-pyrone naturelle ou synthétique étant un composé choisi parmi l'urolithine A, l'isourolithine A, l'urolithine B, l'urolithine C, l'urolithine D, l'urolithine E, l'urolithine M-5, l'urolithine M-6, l'urolithine M-7, l'isourolithine B, l'urolithine A glucuronide, l'urolithine B glucuronide, la 8-Omethylurolithine A, la 8,9-di-O-methylurolithine C, et la 8,9-di-O-methylurolithine D est un composé dont la structure chimique de base est la suivante (Formule générale (II)) : 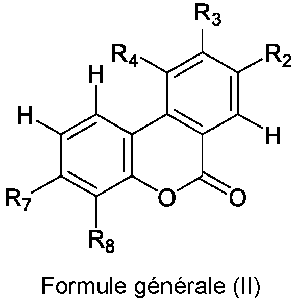 dans laquelle,
R₂ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₃ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₄ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₇ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4 ;
R₈ représente un hydrogène, un alkyle en C₁₋₄, CH(=O), C(=O)OR', OR', OSOsR', C(=O)R', OC(=O)R', un halogène, un O-dihydrobenzofurane, un O-glucoside, un C-glucoside, un O-glucoside acylé, un C-glucoside acylé, un O-glucoside sulfaté, ou un C-glucoside sulfaté; ledit alkyle en C₁₋₄ étant éventuellement substitué par un ou plusieurs hydroxy; ledit O-dihydrobenzofurane étant éventuellement substitué par un ou plusieurs groupements choisi parmi OR' ou CH₂OR'; R' représentant un hydrogène ou un alkyle en C1-4.

6. Composition selon l'une quelconque des revendications précédentes, **caractérisée en ce qu'**elle comprend en outre au moins un agent plastifiant.

7. Composition selon l'une quelconque des revendications précédentes, **caractérisée en ce qu'**elle comprend en outre au moins un additif choisi dans le groupe constitué des agents lubrifiants, des agents surfactants, des agents antioxydants, des agents chélatants, de leurs dérivés et de leurs mélanges.

8. Composition selon l'une quelconque des revendications précédentes, **caractérisée en ce qu'**elle comprend en outre au moins un premier composé additionnel de type polyphénol choisi dans le groupe constitué des acides phénoliques, des stilbènes, des alcools phénoliques, des lignanes, des flavonoïdes, de leurs dérivés et de leurs mélanges.

9. Procédé de fabrication, en particulier procédé de fabrication par thermoformage, d'une composition sous forme d'un extrudat thermoformé selon l'une quelconque des revendications 1 à 8, **caractérisé en ce qu'**il comprend les étapes suivantes :
a) une étape d'amenée simultanée ou différée dans le temps d'au moins une dibenzo-α-pyrone naturelle ou synthétique comme substance active et d'au moins un support naturel ou synthétique, pour alimenter un extrudeur,
b) une étape de mélange, dans ledit extrudeur, de ladite au moins une dibenzo-α-pyrone naturelle ou synthétique comme substance active et dudit au moins un support naturel ou synthétique, pour former un mélange, et
c) une étape d'extrusion à chaud dudit mélange obtenu à l'étape b) dans ledit extrudeur pour obtenir un extrudat thermoformé, en particulier pour obtenir un extrudat thermoformé consistant en une dispersion solide, plus particulièrement pour obtenir un extrudat thermoformé consistant en une dispersion solide consistant en une structure vitreuse comprenant un mélange moléculaire de ladite au moins une dibenzo-α-pyrone naturelle ou synthétique comme substance active et dudit au moins un support naturel ou synthétique.

10. Procédé selon la revendication 9, **caractérisé en ce qu'**il comprend une étape préalable de pré-mélange de ladite au moins une dibenzo-α-pyrone naturelle ou synthétique comme substance active active et dudit au moins un support naturel ou synthétique, de telle sorte à former un pré-mélange destiné à alimenter l'extrudeur.

11. Procédé selon la revendication 9 ou 10, **caractérisé en ce que** ladite étape d'extrusion à chaud est réalisée à une température d'extrusion comprise entre 20 et 300°C.

12. Procédé selon l'une quelconque des revendications 9 à 11, **caractérisé en ce que** ladite étape d'extrusion à chaud est réalisée à une vitesse de rotation d'une vis d'extrusion comprise entre 20 et 900 tours/min, de préférence comprise entre 50 et 300 tours/min, de préférence comprise entre 100 et 250 tours/min, préférentiellement égale à 250 tours/min, préférentiellement égale à 200 tours/min, préférentiellement égale à 100 tours/min.

13. Procédé selon l'une quelconque des revendications 9 à 12, **caractérisé en ce qu'**il comprend une étape additionnelle de refroidissement en sortie de l'extrudeur.

14. Procédé selon l'une quelconque des revendications 9 à 13, **caractérisé en ce qu'**il comprend une étape additionnelle de traitement de l'extrudat thermoformé en sortie de l'extrudeur, par exemple une découpe au niveau d'un pelletiseur et/ou un broyage et/ou un concassage et/ou une réduction en poudre dudit extrudat thermoformé.

15. Composition sous forme d'un extrudat thermoformé, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, selon l'une quelconque des revendications 1 à 8 pour utilisation dans le traitement préventif et/ou curatif, chez l'être humain et/ou chez l'animal, de pathologies en lien avec le système musculaire et neuromusculaire, de pathologies en lien avec la colonne vertébrale et les articulations, de pathologies en lien avec le système cardiovasculaire (ischémie, désordres métaboliques, dyslipidémies...), de pathologies en lien avec le système hormonal, de pathologies en lien avec le système gastro-intestinal (nausées, vomissements, diarrhées, inflammations digestives, ...), de pathologies liées au système rénal (néphropathies, lésions rénales aigues, ... ), de pathologies en lien avec le vieillissement prématuré des cellules, antioxydant, de pathologies en lien avec le système endocrinien (hyperglycémie, ...), de pathologies en lien avec le système immunitaire (maladies inflammatoires, myopathies, arthrite rhumatoïde, sclérose en plaque, ostéoarthrite, fibromyalgie,...), de pathologies en lien avec le système nerveux central (douleur, migraine, épilepsie, maladie de Parkinson, maladie d'Alzheimer, anxiété, dépression,...), des troubles du sommeil, de maladies de la peau, de maladies dues à la présence de microorganismes, des cancers (anti-tumoral, ...), des maladies oculaires (glaucoma, ... ), des maladies du système respiratoire (asthme, ... ) et dans le traitement préventif et/ou curatif du diabète.

16. Composition sous forme d'un extrudat thermoformé, éventuellement conditionné en pellet, en flake, en granulé, en poudre, en comprimé effervescent ou non, en solution injectable ou non, en boisson, en suspension, en gel, en pommade ou encore sous toute autre forme adéquate permettant une administration à un animal ou à un être humain, obtenu suivant le procédé selon l'une quelconque des revendications 9 à 14.
